(19) Europäisches Patentamt
European Patent Office
Office européen des brevets

(11) **EP 4 015 078 A1**

(12) **EUROPEAN PATENT APPLICATION**

(43) Date of publication:
**22.06.2022  Bulletin 2022/25**

(21) Application number: **20214362.4**

(22) Date of filing: **15.12.2020**

(51) International Patent Classification (IPC):
**B01J 23/83** $^{(2006.01)}$ **B01J 35/02** $^{(2006.01)}$
**B01J 37/00** $^{(2006.01)}$ **C07C 5/00** $^{(2006.01)}$

(52) Cooperative Patent Classification (CPC):
(C-Sets available)
**B01J 23/83; B01J 35/023; B01J 35/026;
B01J 37/0009; C07C 5/3335;** C07C 2523/83
(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Clariant International Ltd**
**4132 Muttenz (CH)**
• **Clariant Catalysts (Japan) K.K.**
**Tokyo 113-0021 (JP)**

(72) Inventors:
• **SCHULZ, Felix**
**81373 Muenchen (DE)**

• **SCHWARZER, Hans-Christoph**
**85635 Hoehenkirchen (DE)**
• **KODAKARI, Nobuaki**
**Toyama, 939-2753 (JP)**
• **KURAGUCHI, Yuma**
**Toyama, 939-2753 (JP)**
• **KUSABA, Takashi**
**Toyama, 939-2753 (JP)**

(74) Representative: **Kuba, Stefan**
**Clariant Produkte (Deutschland) GmbH
IPM / Patent & License Management
Arabellastraße 4a
81925 München (DE)**

(54) **PROCESS OF PRODUCING ALKENYLAROMATIC COMPOUND USING DEHYDROGENATION CATALYST**

(57) The invention relates to a process for the production of an alkenylaromatic compound comprising the step of:
- contacting a hydrocarbon stream including an alkylaromatic compound with water vapor in the presence of a dehydrogenation catalyst, suitable for the dehydrogenation of the alkylaromatic compound, in one or more consecutive reactors,
wherein the weight ratio between the water vapor and the hydrocarbon (water/hydrocarbon ratio) is from 0.4 to 1.5, wherein the dehydrogenation catalyst comprises three or more of teeth and a body, such that the cross-section of the dehydrogenation catalyst is a toothed-wheel shape, and wherein the dehydrogenation catalyst comprises, based on the total weight of the dehydrogenation catalyst components as oxides,
- from 30 to 90 weight percent (wt. %) of iron calculated as $Fe_2O_3$,
- from 1 to 50 wt. % of potassium calculated as $K_2O$,
- from 1 to 50 wt. % of cerium calculated as $CeO_2$, and
- from 0.01 to 1 wt. % of yttrium calculated as $Y_2O_3$. Further the invention relates to a dehydrogenation catalyst comprising:

- from 30 to 90 weight percent (wt. %) of iron calculated as $Fe_2O_3$,
- from 1 to 50 wt. % of potassium calculated as $K_2O$,
- from 1 to 50 wt. % of cerium calculated as $CeO_2$, and
- from 0.01 to 1 wt. % of yttrium calculated as $Y_2O_3$,
wherein the wt. % is based on the total weight of the dehydrogenation catalyst components as oxides, wherein the dehydrogenation catalyst comprises at least three teeth and a body, such that the cross-section of the dehydrogenation catalyst is a toothed-wheel shape, and a process of synthesizing such a catalyst.

Figure 1

FIG. 1

**(Cont. next page)**

EP 4 015 078 A1

(52) Cooperative Patent Classification (CPC): (Cont.)

C-Sets
**C07C 5/3335, C07C 15/46**

**Description**

**FIELD OF INVENTION**

The present invention relates to a process of producing an alkenylaromatic compound from an alkylaromatic compound by using a dehydrogenation catalyst.

**TECHNICAL BACKGROUND OF INVENTION**

The dehydrogenation reaction of alkylaromatic compound is an endothermic reaction accompanied by volume expansion. For example, the dehydrogenation reaction of ethylbenzene is represented by reaction formula (1) below and is generally performed in the form of a mixture of ethylbenzene gas and water vapor (steam) under elevated temperature.

$$C_6H_5C_2H_5 \rightarrow C_6H_5C_2H_3 + H_2 \text{ -113 kJ/mol} \qquad (1)$$

As the reaction generates two molecules from one, the thermodynamic equilibrium is only at lower pressure at the right side. Therefore, a lower pressure is desired for this reaction. The reaction is endothermic, which means, that high temperatures are necessary, leading to coke formation as a side reaction. To overcome this problem water is added to the reaction. The high heat capacity of the water helps to transfer the energy for the endothermic reaction and reduces coking. Producing steam is expensive and further, the water partial pressure adds to the overall pressure which should be kept low in order to avoid a detrimental effect on the thermodynamic equilibrium of the reaction. Therefore, during the last years the tendency in the industry was to reduce the water content. The water content in this reaction is normally given in the form of the water to hydrocarbon weight ratio (W/H ratio). The challenge consists in reducing the W/H-ratio meanwhile preventing coking of the catalyst, or in other words, there is the need of more coking resistant catalysts.
Catalysts based on iron oxide with alkali- and earth-alkali-metals and cerium and palladium as promotors were used over a long time for this reaction.
This is for example described in Ertl, Knözinger, Weitkamp, VCH 1997, Vol. 5 page 2151 ff and US 6191065 B1. US 6191065 B1 describes a catalyst for the production of alkenylaromatics from alkylaromatics, wherein the catalyst is predominantly iron oxide, an alkali metal compound and less than about 100 ppm of a source for a noble metal, such as palladium, platinum, ruthenium, rhenium, osmium, rhodium or iridium. Additional components of the catalyst may include compounds based on cerium, molybdenum, tungsten and other such promoters. Also, a process for the production of alkenylaromatics from alkylaromatics using this catalyst is disclosed. A steam to hydrocarbon ratio is given as molar ratio of 12/1, which means a weight ratio of 1.66/1 (Example 2, col. 9, line 19f).
EP 3388147 discloses a dehydrogenation catalyst for alkylaromatic hydrocarbons, with a catalyst comprising iron (Fe), potassium (K), cerium (Ce), yttrium (Y) and palladium (Pd). The dehydrogenation catalyst shows a high activity at low W/H-weight ratios from 0.8 to 1.2.
A high pressure-drop over the reactor is undesirable, because at the entrance a higher pressure is necessary to drive the gas through the reactor and the thermodynamic conditions are worse there. On the other hand, a high void fraction in the reactor reduces the amount of catalyst in the reactor. Therefore, an optimum must be found between the pressure drop and the amount of catalyst within the reactor. One method to reduce pressure drop without reducing too much the amount of active mass is the use of catalysts of a non-cylindrical form with higher void fraction. A successful example is the use of ribbed extruded catalyst in the form of a tooth wheel.
US 5097091 discloses a toothed wheel form of catalyst used at a steam to hydrocarbon ratio weight ratio of 2. This document also discloses an activity index which is calculated from the total area (geometrical surface area) of the molded particle in a liter with the pressure drop (delta p) relative to the molded particle packing in a reactor with a length of 1 m and a diameter of 1 m. If the reaction is so fast, that it takes place only at the outer surface of the particles and not in the pores the activity index correlates to the activity. If the reaction in the pores plays a role, the conversion is related to the catalyst mass. This is normally the case for the dehydrogenation of ethylbenzene. Therefore, the use of such toothed-wheel shape catalysts does not increase the conversion in a reactor under otherwise identical conditions, because of the higher void fraction correlating with the lower catalyst mass in the reactor.
J. Towfighi, et. al. discloses, that basic alkali metals reduce the coke formation in reforming and dehydrogenation reactions. (J. Towfighi, et. al. J. chem. Eng. of Japan (2002), Vol. 35, No. 10 p 923-937). Unfortunately, as the alkali-metal cations are highly soluble in water. The alkali-metal cations are eluted in water vapor and move in the reactor (60) in the direction of the gas flow from the inlet (62) to the outlet (63). The alkali metal migration is one of the main long-term deactivation mechanisms for the dehydrogenation catalyst. This alkali metal migration is for example described in (G.R. Meima et al., Appl. Catal. A: General 212 (2001) 239-245 or W. Beiniasz et. al., Catal. Today 154 (2010) 224-228 or I. Rossetti et. al., Appl. Catal. A: General 292 (2005) 118-123).

This migration of the alkali metals has two negative effects on the catalytic reaction. The first negative effect occurs in the region, where the alkali metal concentration is diminished over time. Here the lower alkali metal concentration changes the composition of the catalyst leading to deactivation as a result of coke formation, and reduced coking resistance. The second negative effect is that the alkali metals will deposit in colder zones near the exit of the reactor and cover the active catalyst.

The extent of these effects strongly depends on the reaction conditions. Most industrial reactors are designed adiabatic, this means, that the temperature decreases over the reactor due to the endothermic reaction, and the temperature at the exit is lower than at the inlet. To drive the reaction closer to equilibrium, often several reactors are placed after each other, with heating of the gas mixture in between. The exact exit temperature of the reactors then strongly depends on the space velocity, the insulation of the reactor and how many steps are used. The redeposition of alkali metals becomes especially a problem at temperatures below 550 °C. Therefore, the problem to be solved was to develop a process of producing an alkenylaromatic compound by dehydrogenation of an alkylaromatic compound which exhibits longer lifetime. In particular, a process for producing an alkenylaromatic compound by dehydrogenation reaction of an alkylaromatic compound shall be provided including an alkali doped catalyst, which shows less alkali metal migration, and allows to work with a low W/H ratio.

## BRIEF DESCRIPTION OF DRAWINGS

Figure 1 is a schematic cross-sectional drawing of a dehydrogenation catalyst as one embodiment.
Figure 2 is a schematic cross-sectional drawing of a dehydrogenation catalyst as another embodiment.
Figure 3 is a schematic cross-sectional drawing of a dehydrogenation catalyst as another embodiment.
Figure 4 is a schematic cross-sectional drawing of a dehydrogenation catalyst as another embodiment.
Figure 5 is a schematic cross-sectional drawing of a dehydrogenation catalyst as another embodiment.
Figure 6 is a schematic drawing of a reactor for a dehydrogenation of alkylaromatic compounds.

## BRIEF SUMMARY OF THE INVENTION

An object of the present invention is to provide an alkali and yttrium doped dehydrogenation catalyst which is highly active in the dehydrogenation reaction of an alkylaromatic compound in the presence of water vapor and which shows a reduced alkali metal migration. As a result of the inventive process a reduced alkali migration can be observed in the high-temperature region (e.g. from 600 to 650 °C) near the inlet of a typical industrial catalyst bed, for the production of an alkenylaromatic compound such as styrene, and also in the low-temperature region (e.g. under 600 °C) near the outlet, where the temperature decreases as due to the endothermicity of the reaction. It is also an object of the invention to provide a process for producing the catalyst to be used in the inventive process and to provide a dehydrogenation process using the catalyst.

The inventive process of producing an alkenylaromatic compound, comprises the step of:

- contacting a hydrocarbon stream including an alkylaromatic compound with water vapor in the presence of a dehydrogenation catalyst, suitable for the dehydrogenation of the alkylaromatic compound, in one or more consecutive reactors,

wherein the weight ratio between the water vapor and the alkylaromatic compound (water/hydrocarbon ratio) is from 0.4 to 1.5,
wherein the dehydrogenation catalyst comprises three or more of teeth and a body, such that the cross-section of the dehydrogenation catalyst is a toothed-wheel shape, and
wherein the dehydrogenation catalyst comprises, based on the total weight of the dehydrogenation catalyst components as oxides,

- from 30 to 90 weight percent (wt. %) of iron calculated as $Fe_2O_3$,
- from 1 to 50 wt. % of potassium calculated as $K_2O$,
- from 1 to 50 wt. % of cerium calculated as $CeO_2$, and
- from 0.01 to 1 wt. % of yttrium calculated as $Y_2O_3$.

Preferably the tooth-wheel shape has the dimensional relationships of:

(i) a ratio of outside diameter ($d_2$) and body diameter ($d_1$) ($d_2$:$d_1$) is from 1.2:1 to 2.5:1,

(ii) a ratio of a root gap between the teeth ($b_1$) and top width of tooth ($b_2$) ($b_1$:$b_2$) is from 0.1:1 to 0.9:1, and
(iii) the root gap between the teeth ($b_1$) is 0.1 mm or more.

Preferably the toothed wheel shape shows the dimensional relationships: (i) a ratio of outside diameter ($d_2$) and body diameter ($d_1$) ($d_2$:$d_1$) from 1.2:1 to 2.5:1, (ii) a ratio of a root gap between the teeth ($b_1$) and top width of tooth ($b_2$) ($b_1$:$b_2$) from 0.1:1 to 0.9:1, and (iii) the root gap between the teeth ($b_1$) of 0.1 mm or more. Another aspect of the invention relates to a process for the production of a dehydrogenation catalyst comprising the steps of:

- mixing a raw material with water to prepare an extrudable mixture wherein the raw material comprises an iron compound, a potassium compound, a cerium compound and an yttrium compound;
- forming the extrudable mixture into a pellet by extrusion with a matrix with holes in the form of toothed-wheel; and
- calcining the pellet.

Surprisingly we found, that the use of such "ribbed" extruded ethylbenzene dehydrogenation catalyst as described above with tooth wheel structure designed for a reaction with a low W/H ratio, can reduce the alkali metal transport in the reactor, if the catalyst contains yttrium (Y) as a promotor.
Furthermore, we found surprisingly, that the reduction of the conversion, which is normally associated with such a ribbed catalyst, due to the higher void fraction, is lower if the catalyst contains yttrium. As a second unexpected effect, it was observed that the conversion of the catalyst according to the invention is higher than of the standard non-yttrium containing ribbed catalyst, which ultimately results in a higher productivity of the catalyst.

## DESCRIPTION OF THE INVENTION

The invention is a process for the production of an alkenylaromatic compound comprising the step of:

- contacting a hydrocarbon stream including an alkylaromatic compound with water vapor in the presence of a dehydrogenation catalyst, suitable for the dehydrogenation of the alkylaromatic compound, in one or more consecutive reactors,

wherein the weight ratio between the water vapor and the alkylaromatic compound (water/hydrocarbon ratio) is from 0.4 to 1.5,
wherein the dehydrogenation catalyst comprises three or more of teeth and a body, such that the cross-section of the dehydrogenation catalyst is a toothed-wheel shape, and wherein the dehydrogenation catalyst comprises, based on the total weight of the dehydrogenation catalyst components as oxides,

- from 30 to 90 weight percent (wt. %) of iron calculated as $Fe_2O_3$,
- from 1 to 50 wt. % of potassium calculated as $K_2O$,
- from 1 to 50 wt. % of cerium calculated as $CeO_2$, and
- from 0.01 to 1 wt. % of yttrium calculated as $Y_2O_3$.

Preferably the tooth-wheel shape has the dimensional relationships of:

(i) a ratio of outside diameter ($d_2$) and body diameter ($d_1$) ($d_2$:$d_1$) is from 1.2:1 to 2.5:1,
(ii) a ratio of a root gap between the teeth ($b_1$) and top width of tooth ($b_2$) ($b_1$:$b_2$) is from 0.1:1 to 0.9:1, and
(iii) the root gap between the teeth ($b_1$) is 0.1 mm or more.

### Toothed-wheel shape

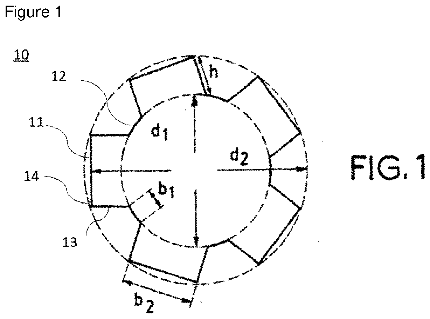

The dehydrogenation catalyst, suitable for the dehydrogenation of alkylaromatic, short the "dehydrogenation catalyst" or "catalyst" (10) comprises teeth (11) and a body (12), which are arranged in a way that the cross-section of the catalyst has the shape of a tooth-wheel. Within this application "cross section" means a cross-section parallel to the plane extended by the teeth of the tooth wheel. A typical example of such a catalyst with body and teeth forming the cross section of a tooth-wheel shape is shown in figure 1.
The body has preferably the shape of a cylinder or a shape being essentially cylindrical. In this case the longitudinal axis, located in the center of the round sides of the cylinder, is perpendicular to the plane extended by the teeth and lies within the center of the tooth wheel (axial direction). Within the cross section the teeth have preferably rectangular shape.
In one embodiment, the dimensional relationships of the toothed-wheel shape are of:

(i) a ratio of outside diameter ($d_2$) and body diameter ($d_1$) ($d_2:d_1$) is from 1.2:1 to 2.5:1;
(ii) a ratio of a root gap between the teeth ($b_1$) and the top width of the tooth ($b_2$) ($b_1:b_2$) is from 0.1:1 to 0.9:1; and
(iii) the root gap between the teeth ($b_1$) is 0.1 mm or more.

In a preferred embodiment, the dimensional relationships of the toothed-wheel shape are of:

(i) a ratio ($d_2:d_1$) was 1.5:1, outside diameter ($d_2$) was 4.6 mm, body diameter ($d_1$) was 3 mm.
(ii) a ratio ($b_1:b_2$) was 0.3:1, a root gap between the teeth ($b_1$) was 0.4 mm, top width of tooth ($b_2$) was 1.5 mm;
(iii) the root gap between the teeth ($b_1$) is 0.4 mm.

The dimensional relationships of the toothed-wheel shape are optimized to increase strength of the catalyst and reduce the catalyst surface contact between the individual catalyst particles in a reactor packing.

The toothed-wheel shape according to the invention can exhibit a ratio of the outside diameter ($d_2$) and the body diameter ($d_1$) ($d_2:d_1$) from 1.3:1 to 2.0:1, preferably from 1.4:1 to 1.8:1, further preferred from 1.5:1 to 1.7:1. The outside diameter ($d_2$) of the tooth-wheel is from 1.5 to 25 mm, preferably from 1.8 to 18 mm further preferred from 2.3 to 12 mm, more preferred from 3 to 9 mm, and most preferably from 3.5 to 6 mm. The body diameter of the tooth-wheel ($d_1$) is from 1 to 10 mm, preferably from 1.5 to 8 mm, further preferred from 1.9 to 6 mm, and most preferably from 2.5 to 4.5 mm.

The dehydrogenation catalyst with the outside diameter ($d_2$) described above is particularly suitable, if the dehydrogenation reactions is carried out under reduced pressure, since a particularly low pressure drop can be achieved this way. On the other hand, the body diameter ($d_1$) described above renders sufficient specific catalyst mass and/or surface area to get sufficient dehydrogenation activity of the catalyst.

The ratio of the root gap between the teeth ($b_1$) and the top width of the tooth ($b_2$) ($b_1:b_2$) in the tooth wheel can be from 0:1 (if there is no root gap between the teeth) to 0.7:1, or it can be from 0.1:1 to 0.7:1, preferably from 0.2:1 to 0.6:1, most preferably from 0.3:1 to 0.5:1. The root gap from the teeth ($b_1$) is from 0.1 to 2 mm, preferably from 0.15 to 1.5 mm, more preferably from 0.2 to 0.9 mm, more preferred from 0.25 to 0.7 mm, and most preferably from 0.3 to 0.5 mm. The top width of the tooth ($b_2$) is from 0.5 to 5 mm, preferably from 0.7 to 3 mm, further preferably from 0.9 to 2.5 mm, and most preferably from 1.2 to 1.9 mm. Preferably in the tooth wheel the top width of the tooth ($b_2$) is larger than the root gap from the teeth ($b_1$).

Preferably the dehydrogenation catalyst has a length (in axial direction) of from 1 to 20 mm, preferably from 2 to 18 mm, and most preferably from 3 to 6 mm. The ratio of the length (in axial direction) of the dehydrogenation catalyst to the outside diameter ($d_2$) is approximately from 0.5:1 to 5:1, and preferably from 1:1 to 3:1.

The dehydrogenation catalyst (10) comprises in general 3 or more of teeth (11), preferably 4 or more teeth, and most preferably 5 or more teeth. The dehydrogenation catalyst (10) preferably comprises 10 or less teeth (11), more preferably 8 or less teeth, most preferably 6 or less teeth. The dehydrogenation catalyst (10) of figure 1 is an embodiment with 5 teeth.

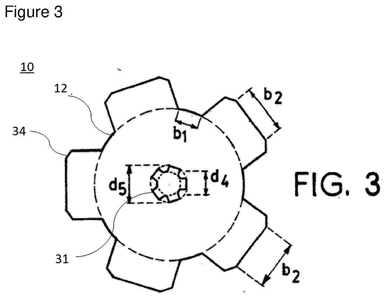

The teeth (11) typically comprises parallel flanks (13). The teeth with flanks partially or fully parallel could reduce catalyst breakage. The tooth flanks (13) run essentially parallel over the tooth height (h). The edges (14) between the tooth top and the tooth flank (13) are right angle. The edges (34) can be chamfered or rounded (Figure 3).

The chamfering or rounding of the edges as shown in Figure 3 also has the advantage that the teeth touch with a catalyst bed or an orderly arrangement on a smaller area, so that a larger exposed surface is available overall. The rounding or chamfering of the teeth can also reduce the abrasion of the catalyst in particular when it is filled into the reactor, or during operation when vibrations or temperature and pressure cycling stresses occur, which lead to a displacement of the shaped bodies relative to one another.

Within the inventive process, the alkylaromatic compound is preferably ethylbenzene, and the alkenylaromatic compound is styrene.

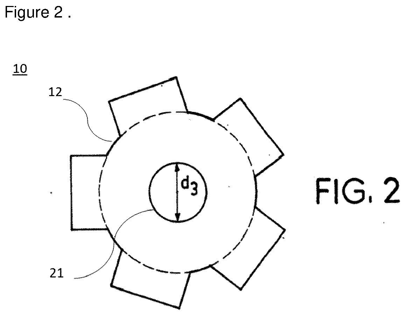

The dehydrogenation catalyst (10) preferably comprises a longitudinal channel (21) in the body (12) (Figure 2). The longitudinal channel (21) is a smooth-cylindrical longitudinal (in axial direction) channel with diameter ($d_3$).

Further, the longitudinal channel (34) has preferably a toothed-wheel shaped cross section with a crown circle diameter ($d_4$) and a root circle diameter ($d_5$) (Figure 3).

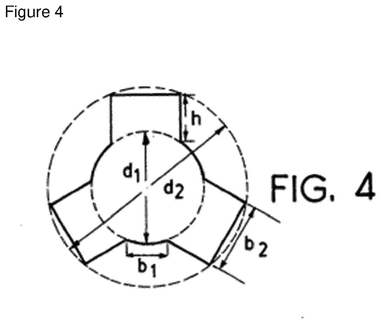

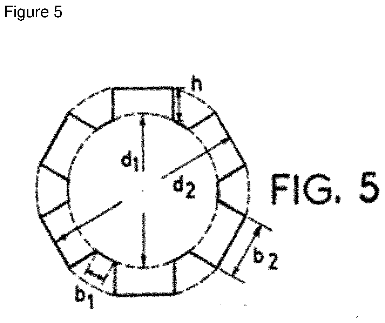

The toothed-wheel shaped cross section comprises 3 teeth as shown in Figure 4 in another embodiment, 6 teeth as shown in Figure 5.

**Process Condition**

The process of producing an alkenylaromatic compound comprises the step of contacting a hydrocarbon stream including or consisting of an alkylaromatic compound with water vapor in the presence of the dehydrogenation catalyst in a reactor.

The dehydrogenation process is conducted as a continuous operation utilizing a fixed bed which may consist of a single reactor. Alternatively, several reactors can be operated at once, in particular the reactors of the dehydrogenation process can be operated as consecutive reactors, which means the outlet of the first reactor is connected to the inlet of the second reactor, and so on.

The inventive process includes the at least partial conversion of a hydrocarbon stream (feed) including an alkylaromatic compound. If the inventive process includes only one reactor, or if the hydrocarbon stream is introduced into the first reactor of a series of consecutive reactors, then the hydrocarbon stream consists, or essentially consists of the alkyl aromatic compound, such as ethylbenzene. In case of the reactor following the first reactor in a series of reactors the hydrocarbon stream includes the alkenyl aromatic compound produced in the preceding reactor as well as unreacted alkyl aromatic compound to be converted.

The weight ratio of the hydrocarbon compounds including, in particular the alkylaromatic compound such as ethylbenzene, and water vapor (water/hydrocarbon ratio) so called W/H ratio is from 0.4 to 1.5. The W/H ratio during the inventive process is generally 0.5 or higher in, preferably 0.6 or higher. The W/H ratio is generally 1.5 or lower, preferably 1.2 or lower, more preferably 1.0 or lower, further preferably 0.9 or lower, and most preferably 0.8 or lower. The water vapor is added to the alkenylaromatic compound feed stock to aid in the removal of carbonaceous residues from the catalyst and to furnish heat for the reaction. The contact time of the reactant-containing gas with the catalyst in a single reactor is expressed in terms of liquid-hourly-space velocity (LHSV). The LHSV is generally from 0.3 to 5 $h^{-1}$, preferably from 0.5 to 3.5 $h^{-1}$, more preferably from 0.6 to 1.9 $h^{-1}$, and most preferably from 0.8 to 1.5 $h^{-1}$.

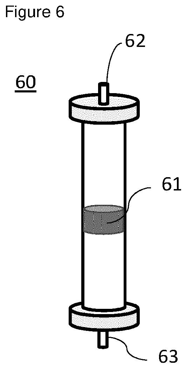

The temperature inside the reactor is from 500 to 700 °C, preferably from 520 to 650 °C. The reactor (60) comprises an inlet (62) and an outlet (63), as shown in Figure 6, whereby the temperature in the inlet is from 550 to 650 °C, preferably from 580 to 630 °C. The temperature in the outlet (63) is lower than the temperature in the inlet (62). The temperature in the outlet (63) is from 500 to 650 °C, preferably from 520 to 600 °C, and most preferably from 530 to 550 °C.

In a preferred embodiment, the reaction is carried out, in a reactor, where the temperature in the exit zone, of the at least one dehydrogenation reactor is below 550°C. In this regard the term "exit zone" means the last 20 cm of the catalyst bed in the reactor in flow direction.

The absolute pressure in the reactor is 150 kPa or lower, preferably 120 kPa or lower, further preferred 110 kPa or lower most preferably 70 kPa. The pressure in the reactor is 1 kPa or higher, preferably 10 kPa or higher, and most preferably 30 kPa or higher.

**Dehydrogenation catalyst composition**

The dehydrogenation catalyst comprises, based on the total weight of the dehydrogenation catalyst, from 30 to 90 weight percent (wt. %) of iron, calculated as $Fe_2O_3$, from 1 to 50 wt. % of potassium calculated as $K_2O$, from 1 to 50 wt. % of cerium, calculated as $CeO_2$, and from 0.01 to 10 wt. % of yttrium, calculated as $Y_2O_3$.

The dehydrogenation catalyst comprises iron (Fe) in the form of an iron compound, whereby the iron compound can be iron oxide and/or composite oxide of iron. Here, "composite oxide" refers to an oxide containing two or more non-oxygen atoms in the structure of the corresponding oxide.

The iron content within the catalyst, calculated as $Fe_2O_3$ is from 40 to 85 wt. % preferably from 45 to 80 wt. %, more preferably from 50 to 81 wt. %, and most preferably from 65 to 75 wt. %, based on the total weight of the dehydrogenation catalyst.

The dehydrogenation catalyst can comprise potassium (K) in the form of a potassium compound. The potassium compound is an oxide and/or a composite oxide of potassium. The Potassium content within the catalyst, calculated as $K_2O$ can be from 5 to 30 wt. %, preferably from 8 to 26 wt. %, more preferably from 10 to 20 wt. % and most preferably from 12 to 18 wt. %, based on the total weight of the dehydrogenation catalyst.

The dehydrogenation catalyst optionally comprises cerium (Ce) in the form of a cerium compound. The cerium compound can be cerium oxide and/or a composite oxide of cerium. The cerium content within the catalyst, calculated as $CeO_2$, is from 3 to 30 wt. %, preferably from 5 to 26 wt. %, more preferably from 7 to 19 wt. % and most preferably from 9 to 14 wt. %, based on the total weight of the dehydrogenation catalyst.

The dehydrogenation catalyst according to the invention comprises yttrium (Y) in the form of an yttrium compound. The yttrium compound can be an yttrium oxide and/or composite oxide of yttrium. The Yttrium content within the catalyst, calculated as $Y_2O_3$ is from 0.01 to 1 wt. %. The Yttrium content within the catalyst, calculated as $Y_2O_3$, is preferably 0.05 wt. % or more, 0.1 wt. % or more, preferably 0.15 wt. % or more, further preferably 0.2 wt. % or more, and most preferably 0.25 wt. % or more, based on the total weight of the dehydrogenation catalyst. The yttrium content within the catalyst, calculated as $Y_2O_3$, is preferably 1 wt. % or less, or 0.8 wt. % or less, further preferably 0.5 wt. % or less, and most preferably 0.3 wt. % or less, based on the total weight of the dehydrogenation catalyst.

The dehydrogenation catalyst according to the invention optionally comprises one or more Group 2 elements selected from the group consisting of calcium (Ca), magnesium (Mg) and a mixture thereof. The group 2 element is preferably calcium (Ca). The Group 2 element calculated, as the oxide of the Group 2 element (XO, with X being the Group 2 element), is present within the catalyst within an amount of 0.3 to 10 wt. %, based on the total weight of the catalyst. The Group 2 element, calculated as the oxide of the Group 2 element, is present within the catalyst typically within an amount of 0.5 wt. % or more, preferably 0.6 wt. % or more, more preferably 0.7 wt. % or more, and most preferably 0.8 wt. % or more, based on the total weight of the dehydrogenation catalyst. The Group 2 element calculated as an oxide of the Group 2 element is typically present within the catalyst within an amount of 8 wt. % or less, preferably 6 wt. % or less, more preferably 3 wt. % or less, most preferably 1.5 wt. % or less, based on the total weight of the dehydrogenation catalyst.

The dehydrogenation catalyst according to the invention optionally comprises a Group 6 element selected from the group consisting of molybdenum (Mo), tungsten (W) and a mixture thereof, preferably the group 6 element is molybdenum (Mo).

The Group 6 element calculated as the oxide of the Group 6 element ($XO_3$ with X being the Group 6 element) is typically present within the catalyst in an amount from 0.1 to 10 wt. %, based on the total weight of the dehydrogenation catalyst. The Group 6 element, calculated as the oxide of the Group 6 element, is present within the catalyst in an amount from 0.2 wt. % or more, preferably 0.3 wt. % or more, more preferably 0.4 wt. % or more, most preferably 0.5 wt. % or more, based on the total weight of the dehydrogenation catalyst. The Group 6 element, calculated as the oxide of the Group 6 element, is typically present within the catalyst in an amount of 8 wt. % or less, preferably 6 wt. % or less, more preferably 3 wt. % or less, most preferably 1.1 wt. % or less, based on the total weight of the dehydrogenation catalyst.

The dehydrogenation catalyst according to the invention optionally comprises sodium (Na) in the form of a sodium compound. The sodium compound is a sodium oxide and/or a composite oxide of sodium. The sodium, calculated as $Na_2O$, can be present within the catalyst in an amount from 0.1 to 15 wt. %, based on the total weight of the dehydrogenation catalyst. The sodium, calculated as $Na_2O$, typically can be present within the catalyst within an amount of 0.5 wt. % or more, preferably 1 wt. % or more, more preferably 1.4 wt. % or more, and most preferably 1.7 wt. %, based on the total weight of the dehydrogenation catalyst. The sodium, calculated as $Na_2O$, is typically present in the catalyst in an amount of 12 wt. % or less, preferably 8 wt. % or less, more preferably, 5 wt. % or less, most preferably 3 wt. % or less, based on the total weight of the dehydrogenation catalyst.

The dehydrogenation catalyst according to the invention optionally comprises a noble metal. The noble metal is selected from the group consisting of gold, silver, platinum, palladium, rhodium, iridium, ruthenium, osmium and a mixture thereof. The noble metal is preferably selected from the group consisting of gold, platinum, palladium and a mixture thereof in another embodiment. The noble metal is most preferably palladium (Pd).

The noble metal is present within the catalyst within an amount from 0.1 to 200 ppm by weight, based on the total weight of the dehydrogenation catalyst. The noble metal can typically be present within an amount of 2 ppm by weight or more, preferably 6 ppm by weight or more, more preferably 10 ppm by weight or more, most preferably 13 ppm by weight or more, based on the total weight of the dehydrogenation catalyst. The noble metal is typically present within the catalyst in an amount of 150 ppm by weight or less, preferably 100 ppm by weight or less, more preferably 80 ppm by weight or less, further preferably 68 ppm by weight or less, even further preferably 42 ppm by weight or less most preferably 25 ppm by weight or less, based on the total weight of the dehydrogenation catalyst.

Most preferably the dehydrogenation catalyst according to the invention consists of from 65 to 80,48 weight percent of iron oxide, from 10 to 20 weight percent of potassium oxide, from 9 to 14 weight percent of cerium oxide, 0.01 to 1 weight percent of yttrium oxide, 0.01 to 1 weight percent of molybdenum oxide, 0.5 to 3 weight percent of CaO, and between 0 to 30 ppm of Palladium, based on the total weight of the dehydrogenation catalyst.

The contents of each element such as iron, potassium, cerium and yttrium and mole ratio among the elements in the catalyst can be determined by using a method known to those skilled in the art, such as elemental analysis with fluorescent X-ray analysis (XRF analysis). For example, the model ZSX Primus II manufactured by Rigaku Corporation can be used for the measurement. First, a sample of dehydrogenation catalyst is ground and then pressed at 20 MPa to make a test sheet approximately with a thickness of approximately 3 mm. Subsequently, the test sheet is subjected to XRF analysis. A calibration curve is separately prepared from the result of the XRF analysis for a standard substance containing an element to be measured, and quantitative calculation is performed with reference to the calibration curve. The quantity of each element thus measured can be appropriately converted to the quantity calculated as the corresponding oxide for example $Fe_2O_3$ for iron and $K_2O$ for potassium or converted to moles to determine the aforementioned contents and mole ratio.

The dehydrogenation catalyst can be a catalyst in the form of a metal oxides. The indications in weight percent are based on the total mass of the catalyst under the assumption that all elements are fully oxidized. The dehydrogenation catalyst can comprise other components other than the components described above. For example, the dehydrogenation catalyst can comprise a binder. Preferably the dehydrogenation catalyst does not comprise chromium (Cr).

**Process of producing catalyst.**

The process of producing the dehydrogenation catalyst comprises steps of:

- mixing a raw material with water to prepare an extrudable mixture;
- forming the extrudable mixture into a pellet by extrusion with a matrix with holes in the form of toothed-wheel;
- and calcining the pellet.

The raw material comprises an iron compound, a potassium compound, a cerium compound and an yttrium oxide.

For a raw material containing iron (i.e. an iron source), the iron compound is an iron oxide, potassium ferrite (a composite oxide of iron and potassium) or sodium ferrite (a composite oxide of iron and sodium) and a mixture thereof or an iron oxide. For the iron oxide, different forms of iron oxide such as red, yellow, brown and black iron oxides can be used. The iron oxide as a raw material is selected from the group consisting of a red iron oxide, a yellow iron oxide, brown iron oxide and black iron oxides and a mixture thereof; a red iron oxide (hematite, $Fe_2O_3$), yellow iron oxide (goethite, $Fe_2O_3.H_2O$) and a mixture thereof; preferably a red iron oxide. The red iron oxide is a hematite with a corresponding crystal structure.

The potassium compound as a raw material is selected from the group consisting of a potassium oxide, a potassium hydroxide, a potassium carbonate, a potassium bicarbonate and a mixture thereof; a potassium carbonate, a potassium hydroxide or a mixture thereof, or a potassium carbonate.

The cerium compound as a raw material is selected from the group consisting of a cerium oxide, cerium hydroxide, cerium carbonate, cerium nitrate, and a mixture thereof; or a cerium carbonate. The cerium carbonate is a cerium carbonate hydrate, cerium hydroxy carbonate or a combination thereof. The cerium carbonate hydrate can comprise 40 wt. % or more of cerium calculated as $CeO_2$, based on the weight of cerium carbonate hydrate. The cerium hydroxy carbonate such as $(CeCO_3OH.xH_2O)$, $(Ce_2(CO_3)_2(OH)_2.H_2O)$, $(Ce(CO_3)_2O.H_2O$, $Ce_2O(CO_3)_2.H_2O$ and $CeO(CO_3)_2.xH_2O)$ are available.

The yttrium compound as a raw material is selected from the group consisting of a an yttrium oxide, a yttrium hydroxide, a yttrium carbonate, a yttrium nitrate, a yttrium phosphate, a yttrium sulfate, a yttrium acetate, a yttrium chloride, a yttrium sulfide and a mixture thereof, or a yttrium oxide, a yttrium nitrate and a mixture thereof, or a preferably yttrium oxide. The yttrium nitrate preferably comprises yttrium nitrate hexahydrate.

If the dehydrogenation catalyst comprises a Group 2 element (Ca), the raw material comprises a compound of the Group 2 element. The compound of the Group 2 element as a raw material is selected from the group consisting of an oxide, a hydroxide, a carbonate, a nitrate, a phosphate, a sulfate, a acetate, a chloride, a sulfide of the Group 2 element and a combination thereof; or an oxide, a hydroxide of the Group 2 element and a combination thereof or preferably a hydroxide of the Group 2 element and a combination thereof.

If the dehydrogenation catalyst comprises a Group 6 element (Mo), the raw material comprises a compound of the Group 6 element. The compound of the Group 6 element as a raw material is selected from the group consisting of an oxide, a salt of an oxoanion of the Group 6 element and a combination thereof, or an oxide of the Group 6 element.

If the dehydrogenation catalyst comprises sodium, the raw material comprises a sodium compound. The sodium compound as a raw material is selected from the group consisting of a sodium oxide, a sodium hydroxide, a sodium carbonate, a sodium nitrate, a sodium phosphate, a sodium sulfate, a sodium acetate, a sodium chloride, a sodium sulfide and a combination thereof, or preferably sodium carbonate.

If the dehydrogenation catalyst comprises a noble metal, the raw material comprises a noble metal compound. The noble metal compound as a raw material is selected from the group consisting of an oxide, a hydroxide, a carbonate, a nitrate, a phosphate, a sulfate, a acetate, a chloride, a sulfide of the noble metal and a combination thereof, or a nitrate of the noble metal.

The raw material and water are mixed to prepare an extrudable mixture. The amount of water is adjustable to be suitable for the subsequent extrusion or to depend on the type of raw materials. Generally, water is present in the extrudable mixture in an amount of from 2 to 50 parts by weight against 100 parts by weight of the raw. The extrudable mixture is formed into a pellet by extrusion with a matrix with holes in the form of toothed wheel. The form of toothed-wheel corresponds with the desired shape of the toothed-wheel as described above.

The extruded pellet of toothed-wheel shape is optionally dried to remove free water. The drying temperature ranges from 60 to 200 °C, preferably from 70 to 150 °C, more preferably from 85 to 110 °C. The drying time is generally from 5 minutes to 5 hours, preferably 10 minutes to 2 hours.

The extruded and dried pellet is calcined. The calcination is carried out to improve the physical stability of the catalyst and improve performance through thermal decomposition of the catalyst precursors. The calcination temperature is from 400 to 1300 °C, preferably from 500 to 1150 °C, most preferably from 800 to 1000 °C. The calcination time is from 30 minutes to 10 hours, preferably from 1 hour to 6 hours, most preferably from 1 hour to 3 hours.

**EXAMPLE**

The present invention is described by the following not limiting examples:

**Comparative example 1 (Y-free and cylindrical shape)**

330 parts by weight of an iron oxide ($Fe_2O_3$), 104.6 parts by weight of potassium carbonate, 17.0 parts by weight of sodium carbonate, 79.8 parts by weight of cerium carbonate hydrate (containing 50% of cerium calculated as the weight of $CeO_2$), 57.0 parts by weight of cerium hydroxy carbonate (containing 70% of cerium calculated as the weight of $CeO_2$) and 6.8 parts by weight of calcium hydroxide and 2.5 parts by weight of molybdenum trioxide were mixed together in a kneader, and aqueous palladium nitrate solution containing 0.008 parts by weight of palladium was added. Further pure water was gradually added to form a paste, and the resulting mixture was shaped in 3 mm cylinders, by extrusion through a matrix with holes of 3 mm diameter. The obtained extrudate was dried at about 90 C, and was then calcined at 900° C for 2 hours. The obtained dehydrogenation catalyst exhibited a cylindrical shape of about 5 mm length, the cross-section of the cylindrical shape was a circle with a diameter of 3 mm.

Alkali metal migration

6 ml of catalyst (61) were diluted with inert ceramic rings 54 ml and placed in the middle of the tube reactor (60). Water and ethylbenzene (EB) were fed into an evaporator prior to the tube reactor (60) and the mixture of evaporated water and EB was then fed into the reactor (60) through the inlet (62). The weight ratio of water to EB (W/H ratio) was 1.0. The liquid hourly space velocity (LHSV) of ethylbenzene in relation to the catalyst bed was 33.3 $h^{-1}$, which accelerated the alkali metal migration. The pressure inside the reactor (60) was 51 kPa (absolute). The temperature in the center of the catalyst bed (61) was controlled to 600 °C by external heaters. The mixture of water vapor and EB vapor was fed to flow down to the outlet (63) over the catalyst bed (61) for one week.

The alkali metal content of the fresh catalyst and the spent catalyst after the one week was analyzed with inductively coupled plasma atomic emission spectroscopy (ICP AES) testing. An alkali metal migration (mg) was then defined as [alkali metal oxide weight (X2O, with X = alkali metal) in fresh catalyst (mg)]-[alkali metal oxide weight in the spent catalyst (mg)].

Styrene monomer yield Productivity

The styrene monomer (SM) yield productivity at 570 °C was calculated by the following equation.

- <u>SM yield Productivity ($g_{SM}/[g_{cat}\cdot h]$)</u>=(EB conversion at 570 °C)x (SM selectivity by weight at 570 °C) x (LHSV: 1.0 $h^{-1}$) x (density of EB: 0.866 g/ml) / (catalyst bulk density, g/ml)

The "SM selectivity by weight at 570 °C" in the equation was the measured SM selectivity when the center of the catalyst bed was controlled to 570 °C. The SM selectivity and EB conversion was measured in the following method. The dehydrogenation reaction was carried out as described above for alkali metal migration except for some different conditions.

The catalyst (61) that was placed in the tube reactor (60) was 100 ml. The weight ratio of water to EB (W/H ratio) was 0.8. The liquid hourly space velocity (LHSV) of ethylbenzene in relation to the catalyst bed was 1.0 $h^{-1}$. The pressure inside the reactor was 101 kPa (absolute pressure). EB vapor ($EB_{inlet}$) with water vapor fed from the inlet (62) flowed down to the outlet (63) over the catalyst bed (61). By external heaters, the temperature at the center of the catalyst bed was controlled to 620 °C for 118 h to reach steady state conditions of the reaction. Thereafter the temperature in the center of the catalyst bed (61) was lowered to 570°C, the resulting dehydrogenated mixture leaving the reactor at the outlet (63) was condensed, the hydrocarbon phase was separated from the water phase and the liquid hydrocarbon phase ($EB_{outlet}$) was analyzed for SM concentration (conc.) and EB concentration (without considering losses to the gas phase, especially hydrogen). The EB conversion and the SM selectivity by weight were determined by the following equations.

SM selectivity=(SM conc. in $EB_{outlet}$−SM conc. in $EB_{inlet}$)/(EB conc. in $EB_{inlet}$−EB conc. in $EB_{outlet}$)

EB conversion=(EB conc. in $EB_{inlet}$−EB conc. in $EB_{outlet}$)/(EB conc. in $EB_{inlet}$)

**Comparative example 2 (Y- free and toothed-wheel shape)**

A dehydrogenation catalyst was made in the same manner as the comparative example 1 except for the shape. The dehydrogenation catalyst was formed by extrusion with a matrix with holes in the form of a toothed-wheel. The dehydrogenation catalyst was 5 mm long and had a cross-section of the toothed-wheel shape as shown in Figure 1 with dimensional relationships of:

(i) a ratio ($d_2$:$d_1$) was 1.5:1, outside diameter ($d_2$) was 4.6 mm, body diameter ($d_1$) was 3 mm.

(ii) a ratio ($b_1$:$b_2$) was 0.3:1, a root gap between the teeth ($b_1$) was 0.4 mm, top width of a tooth ($b_2$) was 1.5 mm;

(iii) the root gap between the teeth ($b_1$) is 0.4 mm.

**Comparative example 3 (Y-containing and cylindrical shape)**

A dehydrogenation catalyst was made in the same manner as the comparative example 1 except for the composition comprising yttrium oxide as shown in Table 1.

**Example 1 (Y-containing and toothed-wheel shape)**

A dehydrogenation catalyst was made in the same manner as the comparative example 2 except for the composition comprising yttrium oxide as shown in Table 1.

Result

Alkali metal migration and SM yield productivity are shown in Table 1. The alkali metal migration rate was 178 mg/week in Comparative Example 2 vs. 275 mg/week in Comparative Example 1 and 200 mg/week in Example 1 vs. 320 mg/week in Comparative Example 3. This shows that the alkali metal migration rate was higher for the Y-containing cylindrical catalyst (comparative example 3) than for the cylindrical Y-free catalyst. This shows the bigger long term stability problem for this catalyst, which shows better fresh performance with low W/H-ratio. On the other hand, this reduction of the alkali metal migration for the examples with toothed-wheel-shape is clear to see.

The SM yield productivity at 570 °C was 0.202 $g_{SM}/[g_{cat}·h]$ in Example 1 and the productivity was 0.185 $[g_{SM}/g_{cat}·h]$ or less in Comparative (Com.) Examples 1 to 3. The SM yield productivity at 570 °C of both cylindrical shaped catalysts (Com. Examples 1 and 3) was 0.174 and 0.178 $g_{SM}/[g_{cat}·h]$. The SM yield productivity at 570 °C of the non-yttrium containing toothed-wheel shaped catalyst Com. Example 2 was 6% higher vs. the non-yttrium containing cylindrical shaped catalyst Com. Example 1. Surprisingly, the relative and absolute increase in SM yield productivity at 570 °C of yttrium containing catalysts by a toothed-wheel shape vs. a cylindrical shape was much larger: The SM yield productivity at 570 °C of Example 1 was 14% higher than of Com. Example 3. As a second unexpected effect, it was observed that the SM yield productivity at 570 °C of Example 1 was 9% higher compared to Com. Example 2. Alkali migration and SM yield productivity are shown in Table 1.

Table 1 (wt. %)

|  | Com. Example 1 | Com. Example 2 | Com. Example 3 | Example 1 |
|---|---|---|---|---|
| $Fe_2O_3$ [wt. %]* | 66.2 | 66.2 | 70.7 | 70.7 |
| $K_2O$ [wt. %]* | 14.3 | 14.3 | 14.3 | 14.3 |
| $Na_2O$ [wt. %]* | 2.0 | 2.0 | 2.0 | 2.0 |
| $CeO_2$ [wt. %]* | 16.0 | 16.0 | 11.0 | 11.0 |
| $Y_2O_3$ [wt. %]* | 0 | 0 | 0.3 | 0.3 |
| $MoO_3$ [wt. %]* | 0.5 | 0.5 | 0.7 | 0.7 |
| CaO [wt. %]* | 1.0 | 1.0 | 1.0 | 1.0 |
| Pd [wt. %]* | 16 ppm | 16 ppm | 16 ppm | 16 ppm |
| Shape | Cylindrical | Toothed-wheel | Cylindrical | Toothed-wheel |
| Catalyst bulk density [g/ml] | 1.51 | 1.28 | 1.53 | 1.30 |

(continued)

|  | Com. Example 1 | Com. Example 2 | Com. Example 3 | Example 1 |
|---|---|---|---|---|
| Alkali metal migration rate [mg/week] | 275 | 178 | 320 | 200 |
| SM yield Productivity at 570 °C [$g_{SM}$/[$g_{cat}$·h]] | 0.174 | 0.185 | 0.178 | 0.202 |
| Selectivity at 570 °C [wt.%] | 98.46 | 98.76 | 98.62 | 98.56 |
| Conv. at 600 °C [%] | 57.7 | 55.3 | 57.7 | 56.3 |
| Conv. at 570 °C [%] | 30.9 | 27.7 | 31.9 | 30.8 |

*based on the total weight of the catalyst components as oxides

**Claims**

1.  A process for the production of an alkenylaromatic compound comprising the step of:

    - contacting a hydrocarbon stream including an alkylaromatic compound with water vapor in the presence of a dehydrogenation catalyst, suitable for the dehydrogenation of the alkylaromatic compound, in one or more consecutive reactors,

    wherein the weight ratio between the water vapor and the hydrocarbon (water/hydrocarbon ratio) is from 0.4 to 1.5, wherein the dehydrogenation catalyst comprises three or more of teeth and a body, such that the cross-section of the dehydrogenation catalyst is a toothed-wheel shape, and
    wherein the dehydrogenation catalyst comprises, based on the total weight of the dehydrogenation catalyst components as oxides,

    - from 30 to 90 weight percent (wt. %) of iron calculated as $Fe_2O_3$,
    - from 1 to 50 wt. % of potassium calculated as $K_2O$,
    - from 1 to 50 wt. % of cerium calculated as $CeO_2$, and
    - from 0.01 to 1 wt. % of yttrium calculated as $Y_2O_3$.

2.  The process of claim 1, wherein the dimensional relationships of the toothed-wheel shape are:

    (i) a ratio of outside diameter ($d_2$) and body diameter ($d_1$) ($d_2$:$d_1$) is from 1.2:1 to 2.5:1;
    (ii) a ratio of a root gap between the teeth ($b_1$) and top width of tooth ($b_2$) ($b_1$:$b_2$) is from 0:1 to 0.9:1;
    (iii) the root gap between the teeth ($b_1$) is 0.1 mm or more.

3.  The process of claims 1 or 2, wherein the outside diameter ($d_2$) is from 3 mm to 10 mm and the body diameter ($d_1$) is from 2 to 8 mm.

4.  The process according to one of the preceding claims, wherein the root gap between the teeth ($b_1$) is from 0.1 to 1 mm and the top width of a tooth ($b_2$) is from 0.8 to 5 mm.

5.  The process according to one of the preceding claims, wherein the dehydrogenation catalyst comprises from 0.3 to 10 wt. % of a Group 2 element, calculated as the oxide of the Group 2 element, based on the total weight of the dehydrogenation catalyst components as oxides, wherein the Group 2 element is selected from the group consisting of calcium (Ca), magnesium (Mg) and a mixture thereof.

6.  The process according to one of the preceding claims, wherein the dehydrogenation catalyst comprises from 0.1 to 10 wt. % of a Group 6 element, calculated as the oxide of the Group 6 element, based on the total weight of the dehydrogenation catalyst components as oxides, wherein the Group 6 element is selected from the group consisting of molybdenum (Mo), tungsten (W) and a mixture thereof.

7.  The process according to one of the preceding claims, wherein the dehydrogenation catalyst comprises from 0.1 to 10 wt. % of an additional alkali metal, calculated as the oxide of the additional alkali metal other than potassium, based on the total weight of the dehydrogenation catalyst components as oxides.

8. The process according to one of the preceding claims, wherein the dehydrogenation catalyst comprises from 0.1 to 200 ppm by weight of one or more noble metal(s) selected from the group consisting of ruthenium (Ru), osmium (Os), iridium (Ir), rhodium (Rh), platinum (Pt), palladium (Pd), silver (Ag) and gold (Au), based on the total weight of the dehydrogenation catalyst components as oxides.

9. A process according to one of the preceding claims, wherein the temperature in the exit zone of at least one reactor, in the last 20 cm of the reactor, is below 550 °C.

10. A dehydrogenation catalyst comprising:

   - from 30 to 90 weight percent (wt. %) of iron calculated as $Fe_2O_3$,
   - from 1 to 50 wt. % of potassium calculated as $K_2O$,
   - from 1 to 50 wt. % of cerium calculated as $CeO_2$, and
   - from 0.01 to 1 wt. % of yttrium calculated as $Y_2O_3$,

   wherein the wt. % is based on the total weight of the dehydrogenation catalyst components as oxides,
   wherein the dehydrogenation catalyst comprises at least three teeth and a body, such that the cross-section of the dehydrogenation catalyst is a toothed-wheel shape.

11. The dehydrogenation catalyst according to claim 10, wherein the tooth-wheel shows the dimensional relationships of:

   - a ratio of outside diameter ($d_2$) and body diameter ($d_1$) ($d_2$:$d_1$) from 1.2:1 to 2.5:1,
   - a ratio of a root gap between the teeth ($b_1$) and top width of a tooth ($b_2$) ($b_1$:$b_2$) from 0.1:1 to 0.9:1, and
   - the root gap between the teeth ($b_1$) is 0.1 mm or more.

12. A process for the synthesis of a dehydrogenation catalyst according to claim 11, comprising the steps of:

   - mixing a raw material with water to prepare an extrudable mixture, wherein the raw material comprises an iron compound, a potassium compound, a cerium compound and an yttrium compound;
   - forming the extrudable mixture into a pellet by extrusion with a matrix with holes in the form of toothed wheel; and
   - calcining the pellet.

Figure 1

FIG. 1

Figure 2 .

FIG. 2

Figure 3

FIG. 3

Figure 4

FIG. 4

Figure 5

FIG. 5

Figure 6

Europäisches Patentamt

European Patent Office

Office européen des brevets

## EUROPEAN SEARCH REPORT

Application Number

EP 20 21 4362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 3 388 147 A1 (CLARIANT CATALYSTS JAPAN KK [JP]) 17 October 2018 (2018-10-17) <br> * abstract * <br> * paragraphs [0015] - [0022], [0105] * <br> * examples 1-3; tables 1-3,5 * <br> * claims 1-27 * <br> ----- | 1-12 | INV. <br> B01J23/83 <br> B01J35/02 <br> B01J37/00 <br> C07C5/00 |
| Y | EP 0 423 694 A1 (SUED CHEMIE AG [DE]) 24 April 1991 (1991-04-24) <br> * abstract; figures 1-5 * <br> * page 2, line 51 - page 3, line 37 * <br> * page 4, line 13 - line 49 * <br> ----- | 1-12 | |

TECHNICAL FIELDS
SEARCHED        (IPC)

B01J
C07C

The present search report has been drawn up for all claims

| Place of search | Date of completion of the search | Examiner |
|---|---|---|
| Munich | 5 May 2021 | Fischbach, Malaika |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT**
**ON EUROPEAN PATENT APPLICATION NO.** EP 20 21 4362

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2021

| Patent document cited in search report | | Publication date | Patent family member(s) | | Publication date |
|---|---|---|---|---|---|
| EP 3388147 | A1 | 17-10-2018 | BR | 112018011542 A2 | 27-11-2018 |
| | | | CA | 3007921 A1 | 15-06-2017 |
| | | | CN | 108430622 A | 21-08-2018 |
| | | | DK | 3388147 T3 | 19-04-2021 |
| | | | EA | 201891389 A1 | 30-11-2018 |
| | | | EP | 3388147 A1 | 17-10-2018 |
| | | | JP | 6648157 B2 | 14-02-2020 |
| | | | JP | WO2017099161 A1 | 04-10-2018 |
| | | | KR | 20180092993 A | 20-08-2018 |
| | | | PH | 12018550077 A1 | 28-01-2019 |
| | | | TW | 201733673 A | 01-10-2017 |
| | | | US | 2018370872 A1 | 27-12-2018 |
| | | | WO | 2017099161 A1 | 15-06-2017 |
| EP 0423694 | A1 | 24-04-1991 | DE | 3935073 A1 | 25-04-1991 |
| | | | EP | 0423694 A1 | 24-04-1991 |
| | | | ES | 2048928 T3 | 01-04-1994 |
| | | | JP | H0639438 B2 | 25-05-1994 |
| | | | JP | H03167140 A | 19-07-1991 |
| | | | KR | 910007841 A | 30-05-1991 |
| | | | US | 5097091 A | 17-03-1992 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6191065 B1
- EP 3388147 A
- US 5097091 A

**Non-patent literature cited in the description**

- **J. TOWFIGHI.** *J. chem. Eng. of Japan,* 2002, vol. 35 (10), 923-937
- **G.R. MEIMA et al.** *Appl. Catal. A: General,* 2001, vol. 212, 239-245
- **W. BEINIASZ.** *Catal. Today,* 2010, vol. 154, 224-228
- **I. ROSSETTI.** *Appl. Catal. A: General,* 2005, vol. 292, 118-123